(19) Europäisches Patentamt / European Patent Office / Office européen des brevets

(11) **EP 3 620 101 B1**

(12) **FASCICULE DE BREVET EUROPEEN**

(45) Date de publication et mention de la délivrance du brevet:
**16.06.2021 Bulletin 2021/24**

(51) Int Cl.:
*A61B 3/00* (2006.01)   *A61B 3/10* (2006.01)
*A61B 3/113* (2006.01)   *G02C 7/02* (2006.01)
*G02C 7/06* (2006.01)

(21) Numéro de dépôt: **19202583.1**

(22) Date de dépôt: **30.10.2014**

(54) **MÉTHODE DE DÉTERMINATION D'AU MOINS UN PARAMÈTRE DE CONCEPTION OPTIQUE POUR UNE LENTILLE OPHTALMIQUE PROGRESSIVE**

BESTIMMUNGSMETHODE MINDESTENS EINER OPTISCHEN KENNGRÖSSE FÜR EINE PROGRESSIVE KONTAKTLINSE

METHOD OF DETERMINING AT LEAST ONE PARAMETER OF OPTICAL DESIGN FOR A MULTIFOCAL OPHTHALMIC LENS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.11.2013 FR 1360990**

(43) Date de publication de la demande:
**11.03.2020 Bulletin 2020/11**

(62) Numéro(s) de document de la (des) demande(s) initiale(s) en application de l'article 76 CBE:
**14825395.8 / 3 065 620**

(73) Titulaire: **Essilor International**
**94220 Charenton-le-Pont (FR)**

(72) Inventeurs:
• **PETIGNAUD, Cécile**
  **94220 Charenton-Le-Pont (FR)**
• **BONNIN, Thierry**
  **94220 Charenton-Le-Pont (FR)**
• **CALIXTE, Laurent**
  **94220 Charenton-Le-Pont (FR)**
• **MARIE, Sarah**
  **94220 Charenton-Le-Pont (FR)**

(74) Mandataire: **Jacobacci Coralis Harle**
**32, rue de l'Arcade**
**75008 Paris (FR)**

(56) Documents cités:
FR-A1- 2 898 993     FR-A1- 2 911 696
FR-A1- 2 924 824     FR-A1- 2 965 364
KR-B1- 101 300 670   US-B2- 6 827 443

**Description**

DOMAINE TECHNIQUE AUQUEL SE RAPPORTE L'INVENTION

La présente invention concerne une méthode de détermination d'au moins un paramètre de conception optique d'une lentille ophtalmique progressive de lunettes de correction visuelle.

Elle concerne en particulier une méthode de détermination d'un paramètre personnalisé de longueur de progression, de déport interne, de profil de progression et/ou d'étendue d'une zone de vision pour une lentille ophtalmique progressive.

Elle concerne, par ailleurs, une méthode de sélection d'une lentille ophtalmique adaptée aux besoins du porteur et à une monture spécifique choisie par un porteur particulier.

ARRIERE-PLAN TECHNOLOGIQUE

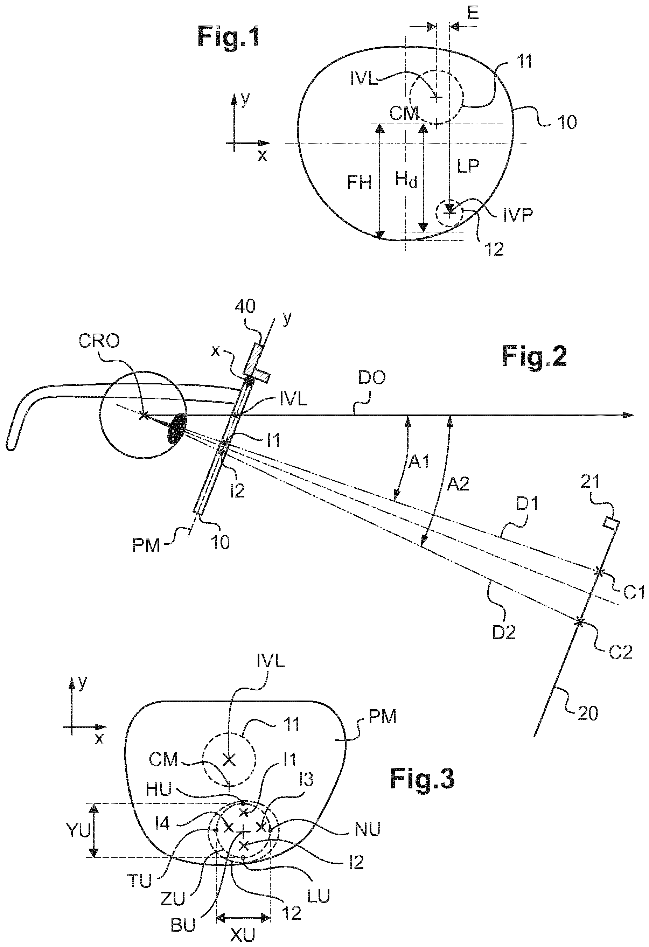

Sur la figure 1, on a représenté schématiquement une lentille ophtalmique progressive 8 en projection dans le plan moyen de la monture 10. On entend par lentille progressive (ou verre progressif) une lentille ophtalmique à addition progressive de puissance sphérique et/ou cylindrique pour lunettes de correction visuelle. La lentille progressive comporte, dans sa partie supérieure, une zone de vision de loin 11 dont la puissance optique est adaptée pour la vision de loin du porteur en fonction de ses besoins de correction visuelle et, dans sa partie inférieure, une zone de vision de près 12 dont la puissance optique est adaptée pour la vision de près de ce porteur.

Entre les zones de vision de loin 11 (ou VL) et de vision de près 12 (ou VP) se trouve, de façon connue, une zone de vision adaptée pour la vision à distance intermédiaire (ou VI, non illustrée).

Le point de référence IVL est un point de vision de loin défini par le fabricant, par exemple au centre d'un cercle délimitant la zone de vision de loin 11. De même, le point de référence IVP est un point de vision de près défini par le fabricant, par exemple au centre d'un cercle délimitant la zone de vision de près 12.

La norme ISO 13666:2012 définit certains paramètres utilisés pour le montage d'une lentille progressive. Ainsi, le point de montage CM est un point situé sur la surface avant d'un verre ou d'un verre semi-fini, que le fabricant considère comme point de référence pour le positionnement du verre devant l'œil. Le point de montage CM est en général repéré par un marquage effaçable qui est retiré après montage. La hauteur de montage FH est la distance verticale qui sépare le point de montage CM de la tangente horizontale passant par le point inférieur de la périphérie de la lentille détourée, c'est-à-dire par le point le plus bas du contour intérieur du cercle de la monture. La hauteur datum (Hd) est la distance verticale entre le point de montage CM et le point de la monture 10 situé à la verticale sous le point de montage CM. La hauteur datum Hd est inférieure ou égale à la hauteur de montage FH, en fonction de la forme de la lentille détourée (qui correspond lorsque la monture est de type cerclée à la forme du contour intérieur du cercle correspondant de la monture 10).

On définit aussi une longueur de progression (LP ou LOC pour length of corridor) comme étant la distance verticale entre la croix de montage et la position du point IVP.

Par ailleurs, on définit le déport interne (ou inset E) de la lentille comme le décalage horizontal entre le point de référence pour la vision de loin IVL et le point de référence pour la vision de près IVP.

La puissance optique de la lentille varie, de préférence continûment, entre lesdits points de référence pour la vision de loin IVL et pour la vision de près IVP, le long d'une ligne (courbe ou brisée) appelée ligne méridienne principale de progression qui passe par ces deux points. Cette ligne méridienne principale de progression traverse les trois zones VL, VI et VP suivant une direction globalement verticale.

La conception d'une lentille progressive vise notamment à déterminer la position des points IVL, IVP, la position et l'étendue de la zone de vision de loin 11, la position et l'étendue de la zone de vision de près 12, la valeur du déport interne E et le profil de progression de l'addition de puissance optique le long de la ligne méridienne principale de progression.

Le choix d'une monture impose certaines contraintes par exemple pour déterminer la longueur de progression Lp.

L'opticien détermine en général les valeurs de longueur de progression et de position des zones VL et VP au cours de mesures d'optique lunetterie, acquises par exemple sur un appareil du type de celui commercialisé sous la marque Visioffice.

Habituellement, le choix de la longueur de progression est fait par l'opticien à partir de critères subjectifs tels que la posture du porteur ou le retour d'expérience de celui-ci sur son équipement précédent.

On connaît également du document US8297752 une méthode pour déterminer la longueur de progression de la lentille selon laquelle on détermine un unique point de vision de loin du porteur et un unique point de vision de près du porteur sur la lentille ophtalmique et on en déduit une longueur de progression correspondante. Une lentille ophtalmique adaptée au porteur peut ainsi être sélectionnée.

Cependant, selon la forme des cercles de la monture, il n'est pas certain, en appliquant cette méthode, que toute la zone pour la vision de près utilisée par le porteur soit inclue dans la lentille ophtalmique une fois celle-ci détourée et montée dans la monture choisie par le porteur.

Le document US2003107707 A1 (ou sa publication US6827443 B2) est un exemple de fabrication d'une lentille progressive prenant en compte le mouvement de la tête du porteur.

OBJET DE L'INVENTION

Afin d'améliorer le confort visuel d'un porteur équipé d'une lentille ophtalmique progressive, il est souhaitable d'ajuster au moins un paramètre de conception optique d'une lentille ophtalmique progressive en fonction du comportement visuel du porteur, de sa posture naturelle pour une tâche visuelle et de la monture choisie par le porteur.

Dans le présent document, on entend par paramètre de conception optique d'une lentille ophtalmique progressive, un ou plusieurs paramètres permettant de déterminer :

- le design optique de la lentille ophtalmique, c'est à dire la répartition spatiale de puissance sphérique et/ou cylindrique sur la lentille. Le design optique recouvre en particulier la délimitation spatiale de la zone de vision de près et de la zone de vision de loin ; et/ou
- la répartition spatiale des déviations prismatiques du verre, susceptible d'affecter la direction naturelle du regard.

Le(s) paramètre(s) de conception optique d'une lentille ophtalmique progressive sont ensuite utilisés pour sélectionner une lentille ophtalmique progressive dans une gamme de lentilles proposées par un fabricant ou pour déterminer la surface d'usinage d'une ou des deux faces de la lentille de manière à fabriquer la lentille ophtalmique progressive adaptée au porteur.

Afin de remédier à l'inconvénient précité de l'état de la technique, la présente invention propose une méthode de détermination d'au moins un paramètre de conception optique pour une lentille ophtalmique progressive destinée à équiper une monture d'un porteur, en fonction du comportement visuel de celui-ci.

Plus particulièrement, on propose selon l'invention une méthode selon la revendication 1, comprenant, entre autres, les étapes suivantes :

a) on collecte une pluralité de mesures comportementales relatives à une pluralité de positions et/ou de directions de regard du porteur pendant une tâche visuelle ;
b) on traite statistiquement ladite pluralité de mesures comportementales pour déterminer une zone d'usure de la surface d'un verre monté sur ladite monture, ladite zone d'usure (ZU) étant représentative d'une distribution spatiale statistique de ladite pluralité de mesures comportementales ;
c) on détermine au moins un paramètre de conception optique pour ladite lentille ophtalmique progressive en fonction d'une position et/ou d'une étendue spatiale de la zone d'usure.

D'autres caractéristiques avantageuses de la méthode de l'invention sont les suivantes :

- ledit au moins un paramètre de conception optique comprend au moins un paramètre de conception optique du design de ladite lentille ophtalmique progressive parmi une gamme réduite de longueur de progression [Lpmin ; Lpmax], une longueur de progression, une hauteur de la zone de vision de près, une largeur de la zone de vision de près, un déport interne de ladite lentille ophtalmique progressive ;
- ledit au moins un paramètre de conception optique comprend un profil de progression de la puissance optique suivant une méridienne entre la zone de vision de loin et la zone de vision de près pour ladite lentille ophtalmique progressive.

De façon avantageuse, la méthode de détermination d'au moins un paramètre de conception optique pour une lentille ophtalmique progressive comprend en outre les étapes suivantes :

b1) on calcule une position du barycentre de la zone d'usure ; et
c1) on détermine une valeur de longueur de progression en fonction de la position du barycentre de la zone d'usure.

Dans un mode de réalisation particulier, on détermine une gamme réduite de longueur de progression et dans un autre mode de réalisation particulier la méthode comprend en outre les étapes suivantes :

b2) on détermine le signe de la différence Δ entre la position verticale du barycentre et une position verticale de référence, correspondant à un angle d'abaissement moyen du regard par rapport à une direction primaire du regard du porteur en vision de loin ; et

c2) on détermine une gamme réduite de longueur de progression ou une valeur de longueur de progression en fonction du signe de la différence Δ.

La méthode selon l'invention comprend en outre les étapes suivantes :

a3) on acquiert une mesure de hauteur de montage pour ladite lentille ophtalmique progressive dans ladite monture ;

b3) on calcule la valeur de la différence Δ entre la position verticale du barycentre (BU) et une position verticale de référence, correspondant à un angle d'abaissement moyen du regard par rapport à une direction primaire du regard du porteur en vision de loin ;

c3) on détermine une valeur de longueur de progression en fonction de ladite mesure de hauteur de montage et de ladite valeur de la différence Δ.

Selon une variante de ce mode de réalisation, à l'étape c3) on détermine une valeur de longueur de progression égale à la hauteur de montage diminuée d'une fonction de correction ε, où ε est une fonction de la différence Δ, de la hauteur de montage, d'une mesure de réfraction oculaire du porteur et/ou de la zone d'usure.

Dans un autre mode de réalisation particulier, la méthode comprend en outre les étapes suivantes :

a4) on acquiert une mesure de hauteur de montage pour ladite lentille ophtalmique progressive dans ladite monture ;

b4) on calcule la valeur de la différence Δ entre la position verticale du barycentre (BU) et une position verticale de référence, correspondant à un angle d'abaissement moyen du regard par rapport à une direction primaire du regard du porteur en vision de loin ;

b5) on calcule au moins une valeur représentative d'une dispersion de la zone d'usure ;

c5) on détermine une valeur de longueur de progression en fonction de ladite mesure de hauteur de montage, de ladite valeur de la différence et/ou de ladite au moins une valeur représentative d'une dispersion de la zone d'usure.

De façon avantageuse, à l'étape c5) on détermine une valeur de longueur de progression égale à la hauteur de montage diminuée d'une fonction de correction ε où ε est une fonction de la différence Δ, de la hauteur de montage et de la dispersion de la zone d'usure.

Dans un autre mode de réalisation particulier, la méthode comprend en outre les étapes suivantes :

b6) on calcule la position d'une limite de la zone d'usure;

c6) on détermine un profil de progression de la puissance optique suivant une méridienne entre la zone de vision de loin et la zone de vision de près en fonction de la position de ladite limite de la zone d'usure.

Dans un autre mode de réalisation particulier, la méthode comprend en outre les étapes suivantes :

b7) on calcule la position d'une limite de la zone d'usure ;

c7) on détermine la valeur du déport interne en fonction en fonction de la position de ladite limite de la zone d'usure.

Dans un autre mode de réalisation particulier, la méthode comprend en outre les étapes suivantes :

b8) on calcule une position du barycentre de la zone d'usure et un étalement vertical de la zone d'usure ;

c8) on détermine la hauteur de la zone de vision de près en fonction de la position du barycentre de la zone d'usure et de l'étalement vertical de la zone d'usure.

Dans un autre mode de réalisation particulier, la méthode comprend en outre les étapes suivantes :

b9) on calcule un étalement horizontal de la zone d'usure ;

c9) on détermine la largeur de la zone de vision de près en fonction de l'étalement horizontal de la zone d'usure.

De façon particulière et avantageuse, on module le paramètre de conception optique en fonction de la valeur de compensation sphérique de la prescription de la lentille ophtalmique progressive et/ou de la valeur de l'addition de puissance optique entre la zone de vision de loin et la zone de vision de près, et/ou en fonction d'une mesure d'un angle d'inclinaison de la tête du porteur.

Dans un autre mode de réalisation particulier, la méthode comprend en outre les étapes suivantes :

d) on fournit une pluralité de valeurs moyennes relatives à une surface d'usure associées à une pluralité de porteurs de référence ;

e) on traite statistiquement ladite pluralité de valeurs moyennes associées à ladite pluralité de porteurs de référence pour déterminer une distribution statistique de ladite pluralité de valeurs moyennes ;

f) on détermine une valeur moyenne relative à la surface d'usure pour un porteur pendant ladite tâche visuelle ;

g) on détermine au moins un paramètre de conception optique pour une lentille ophtalmique progressive pour ledit porteur en fonction de ladite valeur moyenne relative à la surface d'usure pour ledit porteur et de ladite distribution statistique de valeurs moyennes associées à ladite pluralité de porteurs de référence.

La présente description concerne aussi une méthode de sélection d'une lentille ophtalmique progressive destinée à un porteur comprenant les étapes suivantes :

- on détermine au moins un paramètre de conception optique par mise en œuvre d'une méthode de détermination selon l'un des modes de réalisation précédents, et
- on sélectionne, parmi un jeu de lentilles standard, une lentille ophtalmique progressive en fonction du paramètre de conception optique déterminé.

La présente description concerne aussi une méthode de détermination d'une lentille ophtalmique progressive destinée à un porteur comprenant les étapes suivantes:

- on choisit au moins une surface courante d'une lentille ophtalmique;
- on détermine au moins un paramètre de conception optique cible par mise en œuvre d'une méthode de détermination selon l'un des modes de réalisation décrits ;
- on détermine ladite lentille ophtalmique progressive par optimisation, dans les conditions de porté, de la surface courante de ladite lentille ophtalmique en utilisant la cible optique déterminée.

La présente description concerne enfin une méthode de fabrication d'une lentille ophtalmique progressive comprenant les étapes suivantes :

- fourniture d'une lentille initiale ;
- détermination de la lentille ophtalmique progressive par mise en œuvre de la méthode de détermination d'une lentille ophtalmique progressive décrite ci-dessus ;
- usinage de la lentille initiale pour réaliser ladite lentille ophtalmique progressive.

La présente description concerne aussi une lentille ophtalmique progressive personnalisée pour un porteur, ladite lentille ophtalmique progressive ayant au moins un paramètre de conception optique déterminé en fonction d'une zone d'usure par mise en œuvre d'une méthode de détermination selon l'un des modes de réalisation décrits.

DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :

- la figure 1 représente une lentille progressive vue de face et différents paramètres utilisés ;
- la figure 2 représente en vue de côté un exemple de système pour l'acquisition de mesures comportementales sur l'œil d'un porteur lors de certaines tâches visuelles en vision de près ;
- la figure 3 représente graphiquement une zone d'usure ZU sous forme d'une surface en projection dans le plan de la monture ;
- les figures 4A, 4B représentent en projection dans le plan moyen de la monture des mesures comportementales pour deux porteurs différents et une distribution statistique associée respectivement à ces mesures comportementales;
- la figure 5 représente une lentille progressive vue de face et un premier mode de comparaison d'une monture avec un design de verre progressif ;
- la figure 6 représente une première variante du premier mode de réalisation pour déterminer une longueur de progression d'une lentille progressive;
- la figure 7 représente une deuxième variante du premier mode de réalisation pour déterminer une longueur de progression d'une lentille progressive;
- la figure 8 représente une troisième variante du premier mode de réalisation pour déterminer une longueur de progression d'une lentille progressive;

- la figure 9 représente une quatrième variante du premier mode de réalisation pour déterminer une longueur de progression d'une lentille progressive;
- la figure 10 représente une cinquième variante du premier mode de réalisation pour déterminer une longueur de progression d'une lentille progressive;
- la figure 11 représente un deuxième mode de réalisation pour déterminer une longueur de progression d'une lentille progressive ;
- la figure 12 représente un troisième mode de réalisation pour déterminer une longueur de progression d'une lentille progressive ;
- la figure 13 représente un quatrième mode de réalisation pour déterminer une longueur de progression d'une lentille progressive ;
- la figure 14 illustre un mode de réalisation pour déterminer l'étendue de la zone d'usure en VP d'une lentille progressive;
- la figure 15 illustre des exemples de détermination du profil de progression de la puissance optique entre la zone VL et la zone VP ;
- la figure 16 illustre un autre mode de réalisation pour déterminer une longueur de progression d'une lentille progressive en fonction des mesures effectuées sur une population de référence.

Dispositif

Sur la figure 2, on a représenté en vue de côté un système pour l'acquisition de mesures comportementales sur l'œil d'un porteur lors de certaines tâches visuelles en vision de près.

La figure 2 est une représentation en projection dans le plan sagittal du porteur. Le plan de la figure 2 est de préférence un plan vertical.

De préférence, le porteur est équipé d'une monture 10 qu'il a choisie, sans aucune lentille montée à l'intérieur.

On peut envisager de placer une monture autre que celle choisie, présentant de préférence des dimensions supérieures à celle choisie de manière à ce que le regard du porteur ne soit pas contraint par les bords de la monture.

On peut aussi envisager de placer la monture choisie avec des lentilles de présentation, ne présentant aucune puissance, ou avec des lentilles correctrices, par exemple des lentilles similaires à celles que le porteur utilise couramment, des lentilles avec correction unifocale ou des lentilles avec correction progressive.

La monture 10 est de préférence équipée d'un système de repérage 40 destiné à permettre la détermination de la position de la tête du porteur dans l'espace à partir d'une image capturée de la tête du porteur équipée du système de repérage. Ce système de repérage est décrit en détails dans le document FR2914173, page 7, ligne 5 à page 10, ligne 8. Il ne sera donc pas décrit plus en détail ici.

Le système de repérage 40 présente des caractéristiques géométriques bien connues, qui permettent, à partir d'une image capturée de la tête du porteur sur laquelle apparaît ce système de repérage, de déterminer la position de la tête du porteur dans l'espace, dans un référentiel lié à ce dispositif de capture d'image.

Pour l'exécution d'une tâche visuelle, on présente au porteur un support 20 (figure 2) qu'il peut par exemple tenir entre ses mains, et placer de la manière qu'il souhaite par rapport à sa tête.

Ce support 20 est de préférence un support plan comportant une partie d'affichage numérique. Il s'agit par exemple d'une tablette tactile.

Ce support 20 comporte de manière générale une cible C1, C2 que le porteur doit suivre du regard pendant la tâche qui lui est assignée.

La tâche visuelle est par exemple une tâche de poursuite au cours de laquelle le porteur suit des yeux les mouvements de la cible C1, C2.

Afin de permettre la détermination des directions du regard du porteur pendant que celui-ci exécute la tâche visuelle qui lui a été assignée, le support 20 comporte au moins un dispositif de capture d'image 21. Il s'agit de préférence d'une caméra pour réaliser une acquisition vidéo du porteur pendant la tâche visuelle.

La position de la cible C1, C2 que le porteur suit des yeux pendant la tâche visuelle est connue à tout instant par rapport au support 20. Elle est donc connue dans un référentiel lié au dispositif de capture d'image 21.

Ainsi, grâce à cet agencement, la position de la cible C1, C2 que le porteur fixe du regard au moment de la capture d'image est connue dans un référentiel lié au dispositif de capture d'image.

L'appareil de capture d'image 21 permet de réaliser une capture d'image de la tête du porteur pour différentes positions de la cible C1, C2 correspondant à différentes directions D1, D2 de regard.

Avantageusement, l'acquisition d'image peut être réalisée via l'enregistrement d'une vidéo.

Les images de la tête du porteur capturée sont transmises à une unité de traitement informatique qui peut être intégrée au support ou déportée.

Les images capturées peuvent être traitées en temps réel ou après la capture de toutes les images.

Ainsi, l'unité de traitement informatique en déduit la direction du regard D1, D2 du porteur lors de chaque capture d'image comme étant la droite reliant le centre de rotation CRO de l'œil avec la cible C1, C2 dans sa position correspondante pendant la capture d'image.

L'unité de traitement informatique permet ensuite de déterminer la position du point d'intersection I1, I2, I3, I4 de la direction du regard D1, D2 du porteur et d'une surface PM liée à ladite monture 10. Avantageusement, la surface PM est le plan moyen du cercle de la monture.

Plus précisément, l'unité de traitement informatique détermine les coordonnées (x,y) de chaque point d'intersection I1, I2 de la direction de regard D1, D2 et du plan moyen PM du cercle de la monture 10 dans un repère orthonormé (X, Y) de ce plan moyen PM.

La figure 2 illustre un exemple de dispositif adapté pour acquérir un ensemble de mesures de directions du regard en vision de près et pour calculer les positions des points d'intersection I1, I2 de la direction de regard D1, D2 et du plan moyen PM du cercle de la monture 10.

De manière alternative, le dispositif de la figure 2 peut être utilisé pour acquérir un ensemble de mesures d'angle d'abaissement (A1, A2) du regard du porteur entre une direction primaire D0 du regard du porteur en vision de loin et une pluralité de directions de regard D1, respectivement D2 en vision de près pour différentes positions de la cible C1, C2. La mesure de l'angle d'abaissement du regard est prise dans le plan sagittal du porteur (plan de la figure 2), le plan sagittal étant de préférence vertical pendant les mesures.

D'autres dispositifs que le dispositif de la figure 2 peuvent être utilisés pour l'acquisition des mesures comportementales utilisées dans le cadre de la présente invention.

De façon avantageuse, le dispositif de mesure enregistre le temps passé par le porteur dans chaque direction de regard correspondant à une mesure.

Un seul et même dispositif de mesure ou plusieurs dispositifs d'optique lunetterie ou d'oculométrie peuvent être utilisés pour collecter des mesures complémentaires relatives à la posture de tête du porteur et/ou à la posture corporelle du porteur associée à chaque mesure de la pluralité de mesures de direction, de position et/ou d'angle d'abaissement du regard du porteur en vision de près.

Procédé

Etape a)

On acquiert un ensemble de mesures comportementales relatives à un porteur, comprenant une pluralité de mesures de directions du regard, une pluralité de positions de points d'intersection I1, I2 de directions du regard avec un plan ou une surface déterminée et/ou une pluralité de mesures d'angle d'abaissement (A1, A2) du regard du porteur.

Dans un premier exemple, on place le porteur dans une situation dans laquelle il effectue une tâche visuelle, par exemple de poursuite visuelle ou de lecture, dans une posture de vision déterminée, par exemple face à un système pour l'acquisition de mesures comportementales sur l'œil d'un porteur, tel qu'illustré en lien avec la figure 2.

Dans un autre exemple, les mesures comportementales relatives à un porteur peuvent provenir d'un enregistrement informatique.

Dans les exemples décrits plus loin, ladite pluralité de mesures comportementales est relative à la vision de près.

De façon avantageuse, le nombre de mesures de la pluralité de mesures comportementales est suffisant pour constituer un échantillon statistiquement significatif. De préférence, ce nombre de mesures est supérieur ou égal à une ou quelques dizaines ou à quelques centaines.

Comme indiqué plus haut, l'acquisition de l'ensemble de mesures comportementales peut être réalisée par tout système de mesure connu. Ainsi, le système de mesure illustré sur la figure 2 est un exemple, mais un autre système de mesure peut reposer sur l'utilisation d'un dispositif de suivi oculaire temporaire ou permanent permettant d'acquérir des données sur l'œil du porteur lors de certaines tâches visuelles. La manière dont on acquiert les mesures des positions des points d'intersection n'est pas limitative.

De manière complémentaire, on acquiert, en plus des mesures comportementales, des données de prescription de correction ophtalmique liées au porteur, des données de monturisation liées à la monture choisie et/ou des données relatives au profil d'addition de puissance sphérique.

De manière préférée, on acquiert des mesures comportementales relatives à un porteur, des données liées à la monture choisie et des données relatives à la prescription du porteur.

L'ensemble des données de départ comprend par exemple :

1- un ensemble de mesures comportementales, comprenant une pluralité de mesures de directions du regard en vision de près, une pluralité de positions de points d'intersection I1, I2 de directions du regard en vision de près avec un plan ou une surface et/ou une pluralité de mesures d'angle d'abaissement (A1, A2) du regard du porteur en vision de près ;

2- une hauteur de montage (FH),
3- des données relatives à la forme de la monture (extraction de la hauteur de montage Datum Hd),
4- une prescription de correction ophtalmique du porteur,
5- un design du profil d'addition de puissance sphérique retenu.

Cet ensemble de départ est ensuite exploité pendant les étapes b) de traitement pour déterminer une zone d'usure et c) de détermination d'au moins un paramètre de conception optique pour une lentille ophtalmique progressive.

Etape b) traitement statistique

On traite ensuite l'ensemble de mesures comportementales pour déterminer une zone d'usure (ZU) représentative d'une distribution statistique de ladite pluralité de mesures, par exemple en vision de près.

Dans le présent document, on définit une zone d'usure ZU ou zone de parcours d'un verre comme étant une zone de l'espace représentative d'une distribution statistique d'un ensemble de points sur le verre par lesquels passe le regard du porteur lors d'une tâche particulière de vision, pour une utilisation classique ou pour une distance donnée. La zone d'usure ZU peut être définie de manière équivalente soit spatialement, par une distribution statistique de points (I1, I2) sur le verre ou sur un autre plan de projection, soit vectoriellement, par une distribution statistique de directions du regard (D1, D2). De manière alternative et simplifiée, la zone d'usure ZU peut aussi être définie de manière tabulée par une distribution statistique d'angles d'abaissement du regard (A1, A2) dans le plan sagittal du porteur.

Dans un cas particulier, la zone d'usure ZU peut être limitée à la vision de près.

De manière préférée, la zone d'usure ZU est représentée sous forme graphique par une surface (Figure 3) en projection dans le plan de la monture (PM). Dans un autre mode de réalisation, il est possible de prendre un autre plan de projection que celui lié au verre. A titre d'exemple, on peut utiliser un autre plan de projection que celui des points d'intersection I1, I2 sur le verre, comme un plan virtuel quelconque ou le plan de la cible 20.

Plus précisément, on collecte l'ensemble des coordonnées (x, y) des points de mesures comportementales, comprenant la pluralité de positions de points d'intersection (I1, I2, I3, I4) des directions du regard avec le plan de la monture PM ou un autre plan de projection.

A l'étape b), on traite statistiquement l'ensemble des coordonnées (x, y) de la pluralité des points de mesures comportementales de manière à déterminer une distribution au sens statistique des points de mesure. De façon avantageuse, on effectue une étape de traitement préalable, par exemple pour la suppression des points aberrants et/ou la prise en compte de seulement 95% des points mesurés. La distribution statistique comprend par exemple une position moyenne (en x et/ou en y) de l'ensemble de points mesurés, une position du barycentre BU (en x et/ou en y) de l'ensemble de points mesurés, un écart-type horizontal, un écart-type vertical et/ou d'autres paramètres de la distribution statistique des mesures comportementales. Dans un mode de réalisation, le calcul du barycentre correspond au calcul de la position moyenne de l'ensemble de points mesurés, chaque point mesuré étant affecté d'un coefficient de pondération égal à 1. Ainsi, le barycentre représente la moyenne des directions de regard. Dans un autre mode de réalisation, le calcul du barycentre prend en compte un coefficient de pondération associé à chaque point de mesure ; par exemple, le coefficient de pondération associé à un chaque point de mesure peut être un coefficient lié au temps passé par le porteur sur chaque point de mesure. Plus précisément, le temps passé par unité de surface peut être utilisé comme coefficient de pondération pour pondérer la zone d'usure ZU en termes d'étalement. Par exemple, on pondère une surface utile de la zone d'usure, qui correspond par exemple à 80% du temps passé. Ainsi, la surface d'usure est considérablement réduite et n'a donc pas le même impact dans une même équation faisant intervenir la zone d'usure ZU comparée à une zone d'usure de pondération uniforme, la position du barycentre et/ou l'étendue de la zone d'usure étant affectées par les coefficients de pondération.

A partir de cette distribution statistique, on détermine une zone d'usure ZU représentée par exemple par une surface, définie par la position du barycentre BU de l'ensemble de point mesurés en VP, la forme de cette surface, par exemple circulaire, elliptique ou quelconque, l'étendue spatiale dans une ou deux dimensions spatiales XU et/ou YU (en fonction de l'écart-type horizontal et/ou vertical) et/ou par des limites haute HU, basse LU, nasale NU et temporale TU. L'étalement de la zone d'usure représente la surface couverte par les mouvements oculaires lors des mesures comportementales. L'étalement de la zone d'usure est définie par une hauteur YU et une largeur XU, et éventuellement une orientation. La distribution ou répartition temporelle dans la zone d'usure représente le temps passé par le porteur par unité de surface de la zone d'usure. En général, cette distribution ou répartition temporelle est gaussienne et centrée sur le barycentre BU de la zone d'usure ZU. Cependant, dans certains cas particuliers, on observe une distribution ou répartition temporelle bimodale, ayant deux maxima locaux dans la zone d'usure.

L'étape b) permet ainsi de déterminer l'étendue spatiale (forme et dimension) de la zone d'usure par rapport aux points d'intersection : une surface, de forme quelconque, entourant tous les points d'intersection, ou une surface ayant un écart-type suivant deux dimensions.

A titre d'exemple non limitatif, la zone d'usure est représentée par une ellipse contenant un pourcentage prédéterminé de points d'intersection mesurés, de préférence 95% des points d'intersection.

La zone d'usure étant une représentation statistique des mesures, cette zone d'usure ne contient pas nécessairement pas la totalité des mesures.

Le traitement statistique peut permettre d'éliminer des mesures aberrantes.

Dans un exemple de réalisation, le traitement statistique des mesures comportementales permet de déterminer :

- une position (X,Y) du barycentre (BU) de la zone d'usure ZU,
- un étalement vertical (YU) et horizontal (XU) de la zone d'usure (95% des points de mesure), et/ou
- une répartition ou distribution du temps passé par unité de surface dans la zone d'usure.

Dans un autre exemple de réalisation, on utilise une pluralité de mesures de direction du regard D1, D2 du porteur, et on traite statistiquement cette pluralité de mesures de direction du regard pour déterminer une distribution statistique vectorielle de la pluralité de mesures de direction du regard.

Ainsi, le traitement statistique de la pluralité de mesures de direction du regard permet de déterminer une distribution statistique définie par :

- une direction moyenne de regard du porteur,
- une direction barycentrique de regard du porteur,
- un écart-type vertical de direction de regard du porteur, et/ou
- un écart-type horizontal de direction de regard du porteur...

A partir de cette distribution statistique de la pluralité de mesures de direction du regard, on détermine une zone d'usure en deux ou trois dimensions, définie par exemple par :

- une direction moyenne de regard du porteur,
- un écart-type autour de la direction moyenne de regard du porteur,
- une direction haute de regard du porteur,
- une direction basse de regard du porteur,
- une direction de convergence maximale de regard du porteur, et/ou
- une direction de divergence maximale de regard du porteur,
- le temps passé par unité d'angle solide dans la zone d'usure.

Dans une variante, on détermine une projection de la pluralité de mesures de direction du regard D1, D2 du porteur dans le plan de la monture (PM) ou dans un autre plan, ce qui ramène au traitement de la pluralité de points de mesure décrit ci-dessus.

De manière alternative et simplifiée, la pluralité de mesures comportementales comporte une pluralité de mesures d'angle d'abaissement du regard (A1, A2) dans le plan sagittal du porteur. Dans ce cas, le traitement statistique permet de déterminer une distribution statistique de pluralité de mesures d'angle d'abaissement du regard. La zone d'usure ZU peut alors être représentée par un tableau comprenant par exemple une valeur moyenne d'angle d'abaissement du regard, un barycentre d'angle d'abaissement du regard, un écart-type d'angle d'abaissement du regard, une valeur basse et/ou une valeur haute d'angle d'abaissement du regard. Dans ce mode de réalisation simplifié, il n'est ainsi pas nécessaire de tracer graphiquement la zone d'usure.

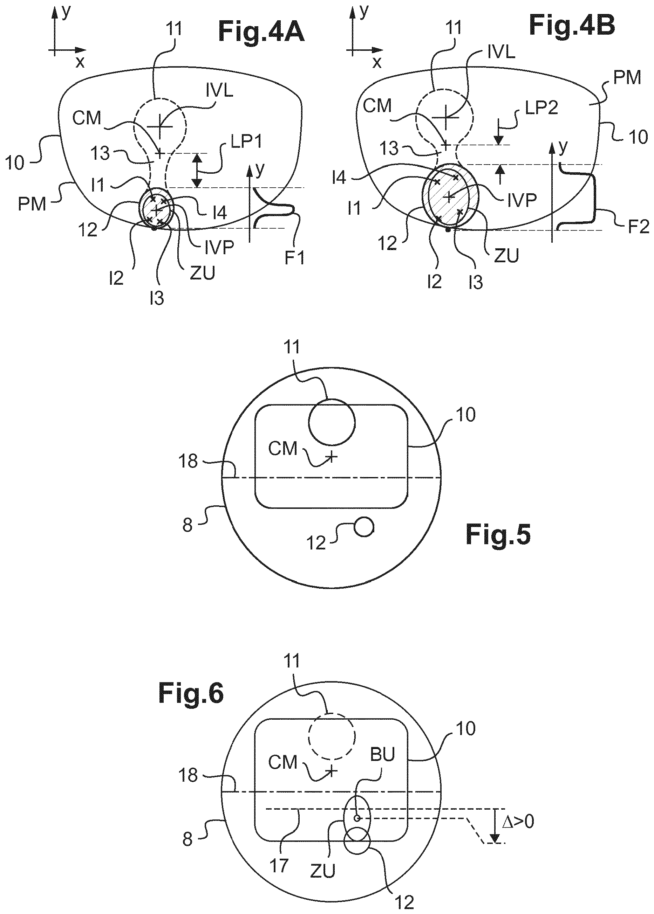

La figure 4A, respectivement 4B, représente un exemple de mesures comportementales des points d'intersection (I1, I2, I3, I4) entre la direction du regard dans le plan moyen de la monture pour un premier porteur, respectivement un second porteur, dans une posture de vision de près.

On note que les éléments identiques ou correspondants des différentes lentilles ophtalmiques représentées en projection dans le plan moyen de la monture sur les figures 4A et 4B sont indiqués par les mêmes signes de référence.

On observe de grandes variations de comportement d'un porteur à un autre pour une même compensation en VL et en VP.

En général, l'abaissement moyen du regard pour passer de la VL à la VP est compris entre 15 et 21 degrés et, de préférence, est de 20 degrés. Cet abaissement moyen du regard correspond à un abaissement moyen confortable du porteur c'est à dire une position de repos pour le regard du porteur. Toutefois, on observe chez certains porteurs un abaissement moyen du regard différent de cette valeur de référence de 20 degrés. Enfin, on observe chez certains porteurs une dispersion spatiale des mesures beaucoup plus importante que chez d'autres porteurs. Cela peut s'expliquer par le fait que, lors d'une même tâche de poursuite visuelle, chaque porteur accompagne les mouvements des yeux par des mouvements de la tête plus ou moins important. Toutefois, il n'est pas indispensable, dans le procédé de l'invention de mesurer les mouvements de tête associés à chaque mesure de position, de direction ou d'abaissement du regard.

Ainsi, sur la figure 4A, on observe que la distribution spatiale des points d'intersection I1, I2, I3, I4 est assez resserrée autour du point IVP en vision de près. Au contraire, sur la figure 4B, on observe que la distribution spatiale des points d'intersection I1, I2, I3, I4 est plus étalée autour du point IVP.

On a représenté sur chaque figure 4A, respectivement 4B, la zone d'usure ZU correspondant au traitement statistique des mesures de points d'intersection (I1, I2, I3, I4) respectivement pour chaque porteur. La zone d'usure est ainsi représentative de la distribution statistique des points mesurés.

Etape c)

Nous allons maintenant décrire comment la distribution statistique des mesures est utilisée pour déterminer au moins un paramètre de conception optique de lentille progressive pour un porteur.

Plus précisément, on cherche à déterminer au moins un paramètre de conception optique parmi : une valeur de longueur de progression, une valeur de déport interne (inset E), une valeur de distance de travail ou une position de confort (ou encore RD pour Reading Distance), et/ou des données d'entrée pour un calcul de design personnalisé.

Sur la figure 4A, respectivement 4B, on note LP1, respectivement LP2, la longueur de progression de la lentille ophtalmique. Dans le cas de la figure 4A, il est préférable de choisir une longueur de progression LP1 supérieure à la longueur de progression LP2 de la figure 4B. Idéalement, la zone d'usure ZU s'étend jusqu'au bord inférieur de la monture, sans intersection avec la monture de manière à éviter une obturation partielle du champ visuel en vision de près.

Procédé - étape c) : premier mode de réalisation de détermination d'une longueur de progression

On définit une ligne de référence 18 qui correspond à un abaissement moyen de direction confortable du regard, de préférence égal à 20 degrés, par rapport à une direction primaire (D0) du regard du porteur en vision de loin.

On calcule une différence Δ entre la ligne de référence 18 et une valeur représentative du comportement visuel du porteur et qui est calculée en fonction :

- de la position du barycentre BU de la zone d'usure ZU et/ou,
- de la position haute HU et/ou basse LU de la zone d'usure ZU.

Dans un mode de réalisation, Δ est définie comme la différence entre la position verticale du barycentre (BU) et une position verticale de référence (17), correspondant à l'angle d'abaissement moyen du regard par rapport à une direction primaire (D0) du regard du porteur en vision de loin.

Le résultat du traitement statistique à l'étape b) donne une indication comportementale, essentiellement liée à une notion de confort, qui peut être traduite au niveau du design par une modulation de la longueur de progression.

On émet une recommandation d'une gamme réduite de longueur de progression Lp (ou LOC pour length of progression) en fonction du signe de Δ.

Par exemple, si Δ est négatif, on préconise une gamme réduite de valeurs de longueur de progression, plutôt courte, de préférence entre 14 et 16mm.

Si Δ est positif, on préconise une gamme réduite de valeurs de longueur de progression, plutôt long, de préférence entre 16 et 18mm.

Procédé : étape c) deuxième mode de réalisation de détermination d'une longueur de progression (Figures 5 à 10)

Dans le deuxième mode de réalisation, on calcule aussi une différence Δ entre la ligne de référence 18 et une valeur représentative du comportement visuel du porteur, la différence Δ étant calculée en fonction de :

- la position du barycentre BU de la zone d'usure ZU, et/ou
- la position haute HU et/ou basse LU de la zone d'usure ZU.

Dans un mode de réalisation, Δ est définie comme la différence entre la position verticale du barycentre (BU) et une position verticale de référence (17), correspondant à l'angle d'abaissement moyen du regard par rapport à une direction primaire (D0) du regard du porteur en vision de loin.

Puis, on ajuste la valeur de longueur de progression par rapport à Δ, de manière à augmenter la part de la zone VP dans le verre en réduisant la valeur de la longueur de progression (LOC), selon une formule du type :

$$LOC = FH - \varepsilon$$

ε représente un paramètre ou une fonction de correction, qui est calculé à partir d'un ou de plusieurs éléments susceptibles d'influencer la valeur du LOC, ces éléments pouvant être de différentes natures :

- en fonction d'un élément matériel qui dépend de la monture et/ou du port de la monture par l'utilisateur tel que la hauteur de montage FH, la hauteur datum (Hd) ou le rectangle inscrit autour de la lentille (ou data boxing) ;
- en fonction de données optiques, telle que la réfraction du porteur, qui permet de prendre en compte des effets prismatiques ;
- en fonction d'aspects comportementaux, tel que l'abaissement du regard, la zone d'usure et le barycentre de la zone d'usure.

Cette liste d'éléments n'est nullement limitative.

Les différentes variantes du deuxième mode de réalisation correspondent à différentes manières de déterminer la valeur du paramètre ε.

Sur la figure 5, on a représenté une lentille progressive 8 non détourée en vue de face. On a aussi représenté une monture 10, ayant une hauteur de montage limitée à moins de 14 mm. La croix de montage est ici placée au centre du rectangle circonscrit autour de la monture 10. La zone VL 11 est située dans la partie supérieure du verre, mais on constate que la zone VP 12 du verre est située en dehors de la monture. Dans ce cas, le résultat du procédé comprend l'émission d'un message d'alerte pour signaler une incompatibilité entre la monture choisie, la mesure, l'ajustage et/ou le design.

Sur les figures 6-7, la monture 10 a une hauteur de montage FH, qui est à titre d'exemple non limitatif, comprise entre 14 mm et 17 mm.

Sur la figure 6, on a représenté le cas où la différence Δ est positive, c'est à dire où l'abaissement moyen du regard du porteur en VP est supérieur à, par exemple, 20 degrés.

Dans cette variante du deuxième mode de réalisation, lorsque la différence Δ est positive, on choisit une valeur de la longueur de progression (LOC), selon une formule du type: LOC = FH

Autrement dit, on fixe la valeur de ε = 0.

Le centre de la zone VP 12 est situé sur le bord de la monture 10 (cf Fig. 6).

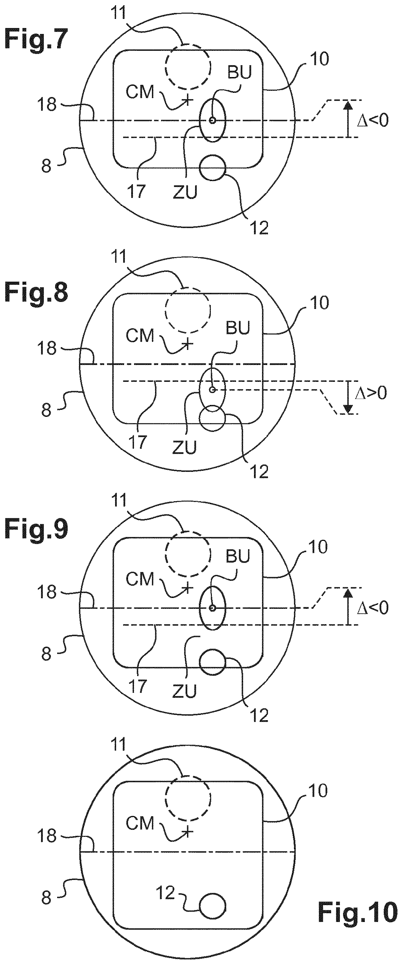

Sur la figure 7, on a représenté le cas où la différence Δ est négative, c'est à dire où l'abaissement moyen du regard du porteur en VP est inférieur à 20 degrés. Dans cette variante du deuxième mode de réalisation, lorsque la différence Δ est négative, on choisit une valeur de longueur de progression (LOC), selon une formule du type :

$$LOC = \max(FH - k(FH); 14mm)$$

$$0 < k(FH) < 1.5mm$$

Dans un exemple non limitatif, la fonction k(FH) est une fonction affine, telle que par exemple : k= 0,35x(FH)-5,25 Dans ce deuxième mode de réalisation, on favorise une longueur de progression dans une gamme longue.

Sur les figures 8-9, la monture 10 a une hauteur de montage FH supérieure à 17 mm.

Dans le cas où la différence Δ est positive (Fig. 8), on choisit une valeur de longueur de progression prédéterminée, par exemple :

LOC = 18 mm

Dans le cas où la différence Δ est négative (Fig. 9), on choisit une valeur de longueur de progression prédéterminée, par exemple :

LOC = 17 mm

La figure 10 illustre le cas où la monture 10 a une hauteur de montage FH supérieure à 20 mm. Dans ce cas, on favorise une longueur de progression dans une gamme longue, de préférence comprise entre 16 et 18 mm.

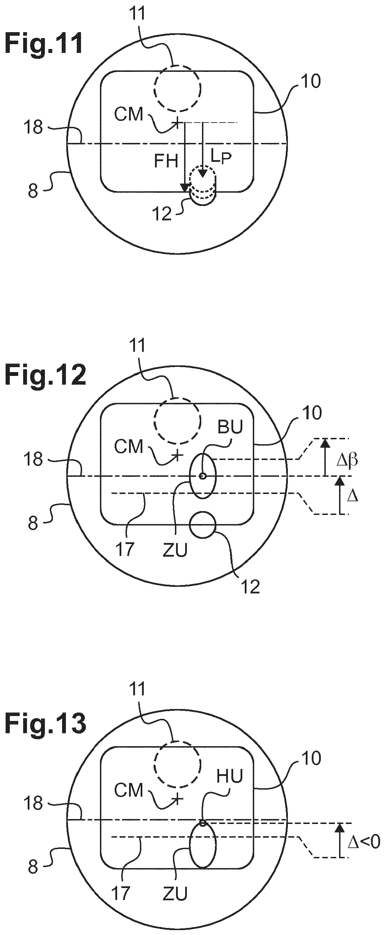

Procédé : étape c) troisième mode de réalisation de détermination d'une longueur de progression (Figure 11)

La figure 11 illustre un troisième mode de réalisation, dans lequel on module la longueur de progression en fonction de la valeur du différentiel Δ entre l'abaissement moyen effectif et un abaissement de regard moyen (typiquement de 20 degrés).

Dans un mode de réalisation, Δ est définie comme la différence entre la position verticale du barycentre (BU) et une position verticale de référence (17), correspondant à l'angle d'abaissement moyen du regard par rapport à une direction primaire (D0) du regard du porteur en vision de loin.

Dans ce cas, on calcule la valeur de la longueur de progression en fonction de la hauteur de montage (FH) et de la valeur de Δ selon une formule du type :

$$LOC = FH - \varepsilon(FH, \Delta)$$

$\varepsilon$ représente un paramètre ou une fonction de correction, qui est calculé à partir d'un ou de plusieurs éléments susceptibles d'influencer la valeur du LOC, ces éléments pouvant être de différentes natures :

- en fonction d'un élément matériel qui dépend de la monture et/ou du port de la monture par l'utilisateur tel que la hauteur de montage FH, la hauteur datum (Hd) ou le rectangle inscrit autour de la lentille (ou data boxing) ;
- en fonction de données optiques, telle que la réfraction du porteur, qui permet de prendre en compte des effets prismatiques ;
- en fonction d'aspects comportementaux, tel que l'abaissement du regard, la zone d'usure et le barycentre de la zone d'usure.

Cette liste d'éléments n'est nullement limitative.

D'un point de vue calcul, le principe est de fixer des bornes sur $\Delta$ et d'affecter des valeurs de correctif optimales :

|  |  | FH |  |
|---|---|---|---|
|  |  | FH min | FH max |
| $\Delta$ |  | 14 | 20 |
| $\Delta$ min | -3 | 0 | 2 |
| $\Delta$ max | 2 | 0 | 3 |

Par exemple, pour FH=14mm on ne tolère aucun raccourcissement quelque soit la valeur de la différence $\Delta$. En revanche pour une hauteur FH=20mm on admet un raccourcissement maximum de 3mm et minimum de 2mm pour respectivement des valeurs de $\Delta$ de 2 et -3 mm.

Ces valeurs, intégrées dans un solveur nous permettent d'obtenir toutes les valeurs intermédiaires par le calcul d'une fonction de transfert du type :

$$\varepsilon = U + V*\Delta + W*FH + X*\Delta*FH$$

où par exemple U= -6,06667 ; V= 60,4667 ; W= 0,43333 ; X= 0,03333
ou

$$\varepsilon = U + V*\Delta + W*FH + X*\Delta*FH + Y*\Delta^2 + Z*FH^2$$

où par exemple U= 5,642 ; V= -0,46667 ; W= -0,984333 ; X= 0,033 ; Y = - 0,00467 ; Z= 0,041667

Les nappes de transfert représentent la fonction de transfert entre l'espace (FH, $\Delta$) et le correctif $\varepsilon$ à appliquer à FH pour obtenir la valeur de longueur de progression :

$$LOC = FH - U + V*\Delta + W*FH + X*\Delta FH \qquad (V1.11)$$

$$LOC = FH - U + V*\Delta + W*FH + X*\Delta*FH + Y*\Delta^2 + Z*FH^2 \qquad (V1.12)$$

La fonction de correction $\varepsilon = k(FH, \Delta)$ peut être une fonction affine, ou plus complexe type quadratique comme illustré ci-dessus, pour une répartition plus homogène de la distribution des valeurs de longueur de progression. Elle peut être également une fonction logarithmique ou exponentielle.

De façon avantageuse, il est en outre possible de moduler la fonction de correction en prenant en compte des paramètres de type correction sphérique Rx , déviation prismatique, design et/ou autre paramètre de type compensation lié à la hauteur de montage datum Hd

Cette modulation peut être faite au niveau de la valeur du LOC ou au niveau de l'expression du paramètre ou de la fonction de correction $\varepsilon$.

Procédé : étape c) quatrième mode de réalisation de détermination d'une longueur de progression (figure 12)

Dans ce quatrième mode de réalisation, on module la longueur de progression en fonction de la différence $\Delta$ entre l'abaissement moyen effectif et l'abaissement de regard moyen (par exemple 20 degrés) et en fonction d'une valeur représentative de la dispersion spatiale ($\Delta\beta$, XU, YU) de la zone d'usure (ZU) (figure 12).

Cette dispersion peut être l'étalement vertical $\Delta\beta$ de la zone d'usure, l'étalement vertical YU de la zone d'usure ou l'étalement horizontal XU de la zone d'usure.

Par exemple, l'étalement vertical $\Delta\beta$ est égal à l'écart-type vertical de la distribution statistique.

Dans un mode de réalisation, $\Delta$ est définie comme la différence entre la position verticale du barycentre (BU) et une position verticale de référence (17), correspondant à l'angle d'abaissement moyen du regard par rapport à une direction primaire (D0) du regard du porteur en vision de loin.

Dans ce cas, on calcule la valeur de la longueur de progression en fonction de la hauteur de montage (FH) et de la valeur de $\Delta$ selon une formule du type :

$$LOC = FH - \varepsilon(FH, \Delta, ZU)$$

Dans une variante de réalisation, on calcule une fonction de transfert telle que:

$$LOC = f(FH, \Delta, \Delta\beta)$$

On fait alors un calcul sur une fonction de transfert du $3^{ème}$ ordre (Figure 12).

La prise en compte de l'étalement de la zone d'usure permet notamment de moduler le LOC en fonction d'un coefficient œil/tête du porteur et plus particulièrement en fonction du comportement œil/tête du porteur, selon que le porteur privilégie les déplacements de la tête plutôt que celui des yeux ou inversement. Cette prise en compte du comportement œil/tête du porteur permet d'assurer un confort visuel optimal pour ce porteur.

De façon avantageuse, il est en outre possible de moduler ces fonctions en prenant en compte des paramètres de type correction sphérique Rx, correction cylindrique, déviation prismatique, design et/ou paramètres type compensation lié à la hauteur de montage datum Hd.

Dans ce cas, la fonction de correction de la longueur de progression s'écrit sous la forme :

$$LOC = FH - \varepsilon(FH, \Delta) - F(RX())$$

où RX représente la réfraction du porteur,
ou encore :

$$LOC = FH - \varepsilon(FH, \Delta, ZU) - C(Boxing/Datum)$$

De manière à effectuer une compensation systématique due au différentiel inhérent entre les mesures de hauteur du rectangle inscrit et de hauteur datum (ou coefficient Boxing/Datum).

Bien sûr, une fonction de correction peut combiner les différents paramètres, par exemple sous la forme :

$$LOC = FH - \varepsilon(FH, \Delta, Usure) - C(Boxing/Datum) - F(RX)$$

Cette modulation peut être faite au niveau de la valeur du LOC ou au niveau de l'expression du paramètre ou de la fonction de correction $\varepsilon$.

De façon avantageuse, il est en outre possible de moduler ces fonctions en prenant en compte des paramètres liés à la posture de tête du porteur et/ou à la posture corporelle du porteur.

Procédé : étape c) cinquième mode de réalisation de détermination d'une longueur de progression (figure 13)

Dans ce cinquième mode de réalisation, on module la longueur de progression en utilisant la différence $\Delta$ entre la borne haute HU de la zone d'usure ZU et l'abaissement de regard moyen (20 degrés) (cf. Figure 13).

Ainsi, l'étalement vertical peut servir à définir une position haute HU permettant de positionner une surface optique ou d'en définir les caractéristiques de design.

De façon alternative, on peut utiliser la valeur de $\Delta$ pour définir des cibles de champ pour le calcul d'un design personnalisé, tel que l'emplacement de la zone VP et l'étendue de la zone VP.

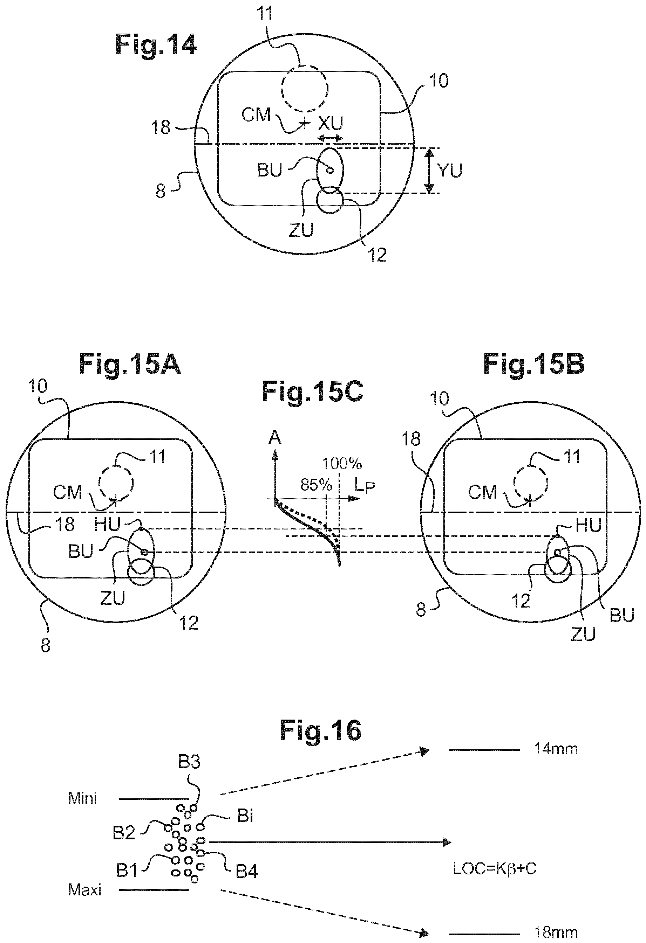

Procédé : étape c) sixième mode de réalisation (figures 14 et 15)

Dans ce sixième mode de réalisation, on utilise les résultats de la distribution statistique pour déterminer ou pour ajuster au moins un paramètre du design optique.

On peut utiliser aussi de manière combinée ou indépendante :

- la position du barycentre BU de la zone d'usure ;
- la limite haute HU de la zone d'usure ;
- la limite basse LU de la zone d'usure ;
- la limite nasale NU de la zone d'usure ;
- la limite temporale TU de la zone d'usure ;
- l'étalement vertical YU de la zone d'usure ;
- l'étalement horizontal XU de la zone d'usure.

Par exemple, on utilise la position en x et/ou en y du barycentre BU de la zone d'usure pour déterminer la position en x et/ou en y du point IVP de la lentille progressive.

Dans un autre exemple, la limite haute HU est utilisée pour ajuster le profil de progression à 85% d'addition de puissance sphérique, qui correspond au début de zone VP, en modifiant le profil de progression du design de manière à fournir une zone VP 12 cohérente avec la stratégie visuelle du porteur (voir figure 15A et 15B). La figure 15C représente deux profils d'addition de puissance optique suivant une ligne méridienne, respectivement en pointillés pour un premier porteur ayant une zone d'usure telle que représentée sur la figure 15A, et respectivement en tirets pour un autre porteur ayant une zone d'usure telle que représentée sur la figure 15B. L'addition totale entre la zone VL et la zone VP est par exemple égale à deux dioptries pour ces deux porteurs. On ajuste le profil de progression de manière personnalisée de manière à ce que la limite haute HU de la zone d'usure corresponde à une addition de puissance sphérique de 85%, c'est-à-dire égale à 1,70 dioptries. De plus, on ajuste la longueur de progression de manière à ce que le barycentre BU corresponde à une addition de 100%, c'est-à-dire fasse partie de la zone de VP.

Dans un autre exemple, l'étalement vertical YU permet d'ajuster la longueur de progression du design de manière à remonter la VP. Cette correction de la longueur de progression est d'autant plus importante que l'étalement vertical YU est grand afin de disposer d'une grande zone VP facilement accessible et adaptée à la stratégie visuelle du porteur.

Dans un autre exemple, la position du barycentre BU et l'étalement vertical YU de la zone d'usure ZU permettent de déterminer la hauteur de la zone de vision de près 12.

Dans un autre exemple, les limites nasale NU et/ou temporale TU permettent de déterminer la valeur du déport E.

On peut aussi utiliser la position Y du barycentre BU comme variable d'ajustement du déport interne E.

Dans un autre exemple, l'étalement horizontal XU permet d'ajuster la largeur de champ du design dans la zone VP, en raccourcissant la longueur de progression, en utilisant des nappes d'ajustement locales et/ou en modifiant directement les cibles en puissance et astigmatisme lors d'une optimisation du design optique.

Ainsi, l'étalement horizontal XU permet de choisir un design plus ou moins doux et étroit ou au contraire dur et large parmi plusieurs design prédéfinis ou dans un continuum.

De façon avantageuse, l'ajustement du design de progression de la lentille progressive prend en compte une mesure de coefficient œil-tête et/ou des données liées à la posture naturelle du porteur afin de lui assurer un confort visuel optimal.

Ainsi, la zone d'usure peut être utilisée comme un paramètre d'entrée afin de venir compléter la fonction de correction.

En plus des paramètres d'origine; tels que la hauteur de montage FH qui rend compte de la monture et de son port, et la différence $\Delta$ qui rend compte de la position de confort du sujet, l'ajout d'une variable de type ZU rend compte de la propension du sujet à explorer l'espace durant la tâche visuelle. Ce mode de réalisation permet de déterminer plus précisément non seulement la longueur de progression (LOC) mais aussi une largeur de champ dans la direction horizontale.

Procédé : septième mode de réalisation (figure 16)

Dans ce mode de réalisation, on collecte un ensemble de distributions statistiques de mesures comportementales de vision relatives à une pluralité de porteurs constituant une population de référence au sens statistique. Cette population de référence peut être segmentée en fonction de l'amétropie, l'âge, le type de porteur ou l'ethnie.

On fournit ainsi une pluralité de valeurs moyennes (de direction moyenne du regard du porteur ou de position moyenne du regard du porteur dans un plan de projection (PM) ou angle moyen d'abaissement du regard du porteur) relatives à une surface d'usure (ZU) associées à la pluralité de porteurs.

Puis, on traite ladite pluralité de valeurs moyennes associées à ladite pluralité de porteurs de référence pour déterminer une distribution statistique de ladite pluralité de valeurs moyennes.

Par exemple, on a représenté sur la figure 16 des points B1, B2, B3, B4 correspondant chacun respectivement, par exemple au barycentre de la surface d'usure pour des mesures comportementales de vision de différents porteurs, telles que décrites aux étapes a) et b).

Pour une population de référence, on effectue un traitement statistique qui permet par exemple de calculer une hauteur maximum et une hauteur minimum.

Dans une étape ultérieure, on détermine une valeur moyenne relative à la surface d'usure (ZU) pour un porteur pendant une tâche visuelle.

Dans l'exemple, le point Bi correspond à la position du barycentre de la zone d'usure pour un porteur dont on cherche à personnaliser la lentille progressive.

On détermine ensuite au moins un paramètre de conception optique pour une lentille ophtalmique progressive pour ledit porteur en fonction de ladite valeur moyenne relative à la surface d'usure pour ledit porteur et de ladite distribution statistique de valeurs moyennes associées à ladite pluralité de porteurs de référence.

Dans l'exemple, on détermine une loi de transfert entre la position du point Bi et la valeur de LOC préconisée.

A titre d'exemple, la loi de transfert est une loi linéaire, selon une formule du type :

$$LOC = K\beta + C$$

Où

$$K = \frac{18-14}{Maxi - Mini}$$

et

$$C = \frac{14.Maxi - 18.Mini}{Maxi - Mini}$$

Ce mode de réalisation permet d'atténuer les erreurs de biais dus à différentes conditions de mesure peu fiables.

De façon avantageuse, les données relatives à la population de référence peuvent être mises à jour en fonction de nouvelles prises de mesure.

Par exemple, un appareil du type de celui commercialisé sous la marque « Visioffice link » peut permettre une mise à jour en ligne des données relatives à la population de référence.

Grâce au procédé de détermination de paramètres de conception optiques de l'invention, on peut déterminer pour un porteur donné la lentille ophtalmique progressive la plus adaptée.

Dans un premier mode de réalisation, cette lentille est sélectionnée en fonction du paramètre de conception optique déterminé parmi un jeu de lentilles standard pour répondre au mieux à ce paramètre de conception optique.

Dans un second mode de réalisation, le procédé de détermination de paramètres de conception optique lentille peut être mis en œuvre dans le cadre d'un procédé d'optimisation de lentille ophtalmique progressive destinée à un porteur de prescription connue. On entend ici par procédé d'optimisation soit un procédé de calcul *ab initio* d'un design de conception optique d'une lentille ophtalmique progressive, soit un procédé de modification d'un design optique existant en fonction du paramètre considéré. Cette modification peut être obtenue via une modification des zones cibles dans un calcul d'optimisation, par ajout direct d'une nappe correctrice à un verre, par étirement de la longueur de progression (LP), ou par choix d'un design parmi différents design de lentilles ophtalmique progressive. Cette optimisation permet de s'approcher au mieux d'une lentille ophtalmique progressive adaptée en fonction des mesures comportementales du porteur.

Ce procédé d'optimisation peut comprendre une première étape de détermination des équations initiales des surfaces avant et arrière de la lentille. Ces déterminations peuvent être réalisées par une lecture de données fournies par le fabricant de la lentille ophtalmique ou par mesure.

Ensuite, au moins une surface courante de lentille ophtalmique est choisie. Cette au moins une surface courante peut être choisie identique à la au moins une surface initiale de la lentille correspondante. Cependant, il est connu de l'homme du métier que cette au moins une surface courante peut également être choisie différente de la au moins une surface initiale de la lentille correspondante. Les surfaces courantes correspondent à une face arrière de la lentille ophtalmique, orientée vers le porteur et/ou à une face avant de la lentille ophtalmique, opposée au porteur.

Puis on détermine une fonction optique cible et des paramètres de conception optique cibles selon le procédé de l'invention pour déterminer, par optimisation, dans les conditions de porté, la surface courante de la lentille en utilisant les cibles optiques déterminées.

Ces cibles fournissent des valeurs de puissance, de module d'astigmatisme et d'axe d'astigmatisme pour des directions de regard données.

A titre d'exemple, on peut déterminer comme cibles pour l'optimisation, une cible de variation de la puissance le long de la méridienne et en particulier une valeur de longueur de progression le long de la méridienne déterminée selon le procédé de l'invention.

La lentille selon l'invention, ainsi obtenue, répond donc mieux aux besoins du porteur et lui apporte un meilleur confort visuel.

De façon avantageuse, il peut être envisagé de renseigner certains paramètres de conception optique déterminés par le procédé selon l'invention sur le bon de commande d'une paire de lentilles ophtalmiques utilisable par un opticien. Les paramètres de conception optique sont associés aux données de puissance prescrite en vision de loin et en vision de près, d'addition prescrite, des écarts pupillaires du porteur, de l'angle pantoscopique de la monture, de la distance verre-œil et des données de la monture choisie par le porteur.

Pour toute case non remplie du bon de commande, la valeur standard moyenne utilisée dans les programmes de calculs existant est retenue.

Chaque exemplaire de bon de commande renseigne un programme informatique de calcul d'optimisation optique afin de calculer la paire de lentilles et d'éditer l'ordre de fabrication qui commande la machine à usinage.

Par ailleurs, ce procédé d'optimisation peut être mis en œuvre dans un procédé de fabrication d'une lentille ophtalmique.

Le procédé de fabrication d'une lentille ophtalmique comprend une première étape de fourniture d'une lentille ophtalmique initiale, lentille semi-finie ou non.

L'étape de détermination de la surface optimisée pour la lentille ophtalmique au moyen du procédé d'optimisation est suivie d'une étape d'usinage de la lentille pour réaliser la au moins une surface optimisée.

Comme indiqué ci-avant, il est possible d'optimiser une ou les deux surfaces de la lentille en fonction des cas. De même, le procédé de fabrication peut être mis en œuvre en usinant une ou les deux surfaces de la lentille ophtalmique initialement fournie. L'usinage des lentilles pour réaliser la ou les surfaces optimisées peut notamment être réalisé par un procédé dit de surfaçage numérique (ou Digital Surfacing) sur une machine-outil à commande numérique adaptée pour l'usinage simple face ou double face d'une lentille ophtalmique progressive.

**Revendications**

1.  Méthode de détermination d'au moins un paramètre de conception optique pour une lentille ophtalmique progressive destinée à équiper une monture (10) d'un porteur, en fonction du comportement visuel de celui-ci, ledit au moins un paramètre de conception optique comprenant une longueur de progression (Lp) de ladite lentille ophtalmique progressive, la méthode comprenant les étapes suivantes :

    a) on acquiert des données liées à la monture choisie et des données relatives à la prescription du porteur et on collecte une pluralité de mesures comportementales relatives à une pluralité de positions et/ou de directions de regard du porteur pendant une tâche visuelle ; et a3) on acquiert une mesure de hauteur de montage (FH, Hd) pour ladite lentille ophtalmique progressive dans ladite monture (10) ;
    b) on traite statistiquement ladite pluralité de mesures comportementales pour déterminer une zone d'usure (ZU) de la surface d'un verre monté sur ladite monture, ladite zone d'usure (ZU) étant représentative d'une distribution spatiale statistique de ladite pluralité de mesures comportementales ; b1) on calcule une position verticale du barycentre (BU) ou d'une borne haute (HU) de la zone d'usure (ZU) et b3) on calcule une valeur d'une différence $\Delta$ entre la position verticale du barycentre (BU) ou de la borne haute (HU) et une position verticale de référence (17), correspondant à un angle d'abaissement moyen du regard par rapport à une direction primaire (D0) du regard du porteur en vision de loin ;
    c) on détermine au moins un paramètre de conception optique comprenant une valeur de longueur de progression (Lp) pour ladite lentille ophtalmique progressive en fonction de ladite mesure de hauteur de montage (FH, Hd) et de ladite valeur de la différence $\Delta$.

2.  Méthode de détermination d'au moins un paramètre de conception optique pour une lentille ophtalmique progressive selon la revendication 1, dans laquelle ledit au moins un paramètre de conception optique comprend au moins un paramètre de conception optique du design de ladite lentille ophtalmique progressive parmi une gamme réduite de longueur de progression [Lpmin ; Lpmax], une hauteur de la zone de vision de près (12), une largeur de la zone de vision de près (12), un déport interne (E) de ladite lentille ophtalmique progressive.

3. Méthode de détermination d'au moins un paramètre de conception optique pour une lentille ophtalmique progressive selon la revendication 1 ou 2, dans laquelle ledit au moins un paramètre de conception optique comprend un profil de progression de la puissance optique suivant une méridienne entre la zone de vision de loin (11) et la zone de vision de près (12) pour ladite lentille ophtalmique progressive.

4. Méthode de détermination d'au moins un paramètre de conception optique pour une lentille ophtalmique progressive selon la revendication 2, comprenant en outre les étapes suivantes :
c1) on détermine une gamme réduite de longueur de progression [Lpmin ; Lpmax] en fonction de la position du barycentre (BU) de la zone d'usure (ZU).

5. Méthode de détermination d'au moins un paramètre de conception optique pour une lentille ophtalmique progressive selon la revendication 4, comprenant en outre les étapes suivantes :

b2) on détermine le signe de la différence $\Delta$ entre la position verticale du barycentre (BU) et une position verticale de référence (17), correspondant à un angle d'abaissement moyen du regard par rapport à une direction primaire (D0) du regard du porteur en vision de loin ; et
c2) on détermine une gamme réduite de longueur de progression [Lpmin ; Lpmax] en fonction du signe de la différence $\Delta$.

6. Méthode de détermination d'au moins un paramètre de conception optique pour une lentille ophtalmique progressive selon la revendication 1, dans laquelle à l'étape c3) on détermine une valeur de longueur de progression (Lp) égale à la hauteur de montage (FH, Hd) diminuée d'une quantité $\epsilon$ où $\epsilon$ est une fonction de la différence $\Delta$, de la hauteur de montage (FH, Hd), d'une mesure de réfraction oculaire du porteur (Rx) et/ou de la zone d'usure (ZU).

7. Méthode de détermination d'au moins un paramètre de conception optique pour une lentille ophtalmique progressive selon la revendication 1, comprenant en outre les étapes suivantes :

b5) on calcule au moins une valeur représentative d'une dispersion ($\Delta\beta$, XU, YU) de la zone d'usure (ZU) ;
c5) on détermine une valeur de longueur de progression (Lp) en fonction de ladite mesure de hauteur de montage (FH, Hd), de ladite valeur de la différence $\Delta$ et de ladite au moins une valeur représentative d'une dispersion ($\Delta\beta$, XU, YU) de la zone d'usure (ZU).

8. Méthode de détermination d'au moins un paramètre de conception optique pour une lentille ophtalmique progressive selon la revendication 7, dans laquelle à l'étape c5) on détermine une valeur de longueur de progression (Lp) égale à la hauteur de montage (FH, Hd) diminuée d'une fonction de correction $\epsilon$ où $\epsilon$ est une fonction de la différence $\Delta$, de la hauteur de montage (FH, Hd) et de la dispersion de la zone d'usure (ZU).

9. Méthode de détermination d'au moins un paramètre de conception optique pour une lentille ophtalmique progressive selon la revendication 3, comprenant en outre les étapes suivantes :

b6) on calcule la position d'une limite (HU, LU, NU, TU) de la zone d'usure (ZU) ;
c6) on détermine un profil de progression de la puissance optique suivant une méridienne entre la zone de vision de loin (11) et la zone de vision de près (12) en fonction de la position de ladite limite (HU, LU, NU, TU) de la zone d'usure (ZU).

10. Méthode de détermination d'au moins un paramètre de conception optique pour une lentille ophtalmique progressive selon l'une des revendications 2 à 9, comprenant en outre les étapes suivantes :

b7) on calcule la position d'une limite (NU, TU) de la zone d'usure (ZU);
c7) on détermine la valeur du déport interne (E) en fonction de la position de ladite limite (NU, TU) de la zone d'usure (ZU).

11. Méthode de détermination d'au moins un paramètre de conception optique pour une lentille ophtalmique progressive selon la revendication 2, comprenant en outre les étapes suivantes :

b8) on calcule un étalement vertical (YU) de la zone d'usure (ZU) ;

c8) on détermine la hauteur de la zone de vision de près (12) en fonction de la position du barycentre (BU) de la zone d'usure (ZU) et de l'étalement vertical (YU) de la zone d'usure (ZU).

**12.** Méthode de détermination d'au moins un paramètre de conception optique pour une lentille ophtalmique progressive selon l'une des revendications 2 ou 11, comprenant en outre les étapes suivantes :

b9) on calcule un étalement horizontal (XU) de la zone d'usure (ZU) ;

c9) on détermine la largeur de la zone de vision de près (12) en fonction de l'étalement horizontal (XU) de la zone d'usure (ZU).

**13.** Méthode de détermination d'au moins un paramètre de conception optique pour une lentille ophtalmique progressive selon l'une des revendications 1 à 12, dans laquelle on module le paramètre de conception optique en fonction de la valeur de compensation sphérique (Rx) de la prescription de la lentille ophtalmique progressive et/ou de la valeur de l'addition de puissance optique entre la zone de vision de loin (11) et la zone de vision de près (12).

**14.** Méthode de détermination d'au moins un paramètre de conception optique pour une lentille ophtalmique progressive selon l'une des revendications 1 à 13, dans laquelle on module le paramètre de conception optique en fonction d'une mesure d'un angle d'inclinaison de la tête du porteur.

**15.** Méthode de détermination d'au moins un paramètre de conception optique pour une lentille ophtalmique progressive selon l'une des revendications 1 à 14, dans laquelle :

d) on fournit une pluralité de valeurs moyennes relatives à une surface d'usure (ZU) associées à une pluralité de porteurs de référence ;

e) on traite statistiquement ladite pluralité de valeurs moyennes associées à ladite pluralité de porteurs de référence pour déterminer une distribution statistique de ladite pluralité de valeurs moyennes ;

f) on détermine une valeur moyenne relative à la surface d'usure (ZU) pour un porteur pendant ladite tâche visuelle ;

g) on détermine au moins un paramètre de conception optique pour une lentille ophtalmique progressive pour ledit porteur en fonction de ladite valeur moyenne relative à la surface d'usure pour ledit porteur et de ladite distribution statistique de valeurs moyennes associées à ladite pluralité de porteurs de référence.

**Patentansprüche**

**1.** Verfahren zur Bestimmung mindestens eines optischen Konzeptionsparameters für eine ophthalmische Gleitsichtlinse, die dazu bestimmt ist, ein Gestell (10) eines Trägers zu bestücken, abhängig von dessen Sehverhalten, wobei der mindestens eine optische Konzeptionsparameter eine Progressionslänge (Lp) der ophthalmischen Gleitsichtlinse enthält, wobei das Verfahren die folgenden Schritte enthält:

a) mit dem gewählten Gestell verbundene Daten und Daten bezüglich des Rezepts des Trägers werden erfasst, und eine Vielzahl von Verhaltensmesswerten bezüglich einer Vielzahl von Positionen und/oder Blickrichtungen des Trägers während einer Sehaufgabe wird gesammelt; und a3) ein Messwert der Montagehöhe (FH, Hd) für die ophthalmische Gleitsichtlinse im Gestell (10) wird erfasst;

b) die Vielzahl von Verhaltensmesswerten wird statistisch behandelt, um eine Abnutzungszone (ZU) der Fläche eines auf das Gestell montierten Glases zu bestimmen, wobei die Abnutzungszone (ZU) für eine statistische räumliche Verteilung der Vielzahl von Verhaltensmesswerten repräsentativ ist; b1) eine senkrechte Position des Schwerpunkts (BU) oder einer oberen Grenze (HU) der Abnutzungszone (ZU) wird berechnet und b3) ein Wert einer Differenz $\Delta$ zwischen der senkrechten Position des Schwerpunkts (BU) oder der oberen Grenze (HU) und einer senkrechten Bezugsposition (17) wird berechnet, die einem mittleren Senkungswinkel des Blicks bezüglich einer primären Richtung (D0) des Blicks des Trägers in Fernsicht entspricht;

c) mindestens ein optischer Konzeptionsparameter, der einen Progressionslängenwert (Lp) für die ophthalmische Gleitsichtlinse enthält, wird abhängig vom Montagehöhe-Messwert (FH, Hd) und vom Wert der Differenz $\Delta$ berechnet.

**2.** Verfahren zur Bestimmung mindestens eines optischen Konzeptionsparameters für eine ophthalmische Gleitsichtlinse nach Anspruch 1, wobei der mindestens eine optische Konzeptionsparameter mindestens einen optischen Konzeptionsparameter des Designs der ophthalmischen Gleitsichtlinse unter einem reduzierten Progressionslängenbereich [Lpmin; Lpmax], einer Höhe der Nahsichtzone (12), einer Breite der Nahsichtzone (12), einem inneren Versatz (E) der ophthalmische Gleitsichtlinse enthält.

3. Verfahren zur Bestimmung mindestens eines optischen Konzeptionsparameters für eine ophthalmische Gleitsichtlinse nach Anspruch 1 oder 2, wobei der mindestens eine optische Konzeptionsparameter ein Progressionsprofil der optischen Leistung gemäß einem Meridian zwischen der Fernsichtzone (11) und der Nahsichtzone (12) für die ophthalmische Gleitsichtlinse enthält.

4. Verfahren zur Bestimmung mindestens eines optischen Konzeptionsparameters für eine ophthalmische Gleitsichtlinse nach Anspruch 2, das außerdem die folgenden Schritte enthält:
c1) ein reduzierter Progressionslängenbereich [Lpmin; Lpmax] wird abhängig von der Position des Schwerpunkts (BU) der Abnutzungszone (ZU) bestimmt.

5. Verfahren zur Bestimmung mindestens eines optischen Konzeptionsparameters für eine ophthalmische Gleitsichtlinse nach Anspruch 4, das außerdem die folgenden Schritte enthält:

b2) das Vorzeichen der Differenz $\Delta$ zwischen der senkrechten Position des Schwerpunkts (BU) und einer senkrechten Bezugsposition (17) wird bestimmt, die einem mittleren Senkungswinkel des Blicks bezüglich einer primären Richtung (D0) des Blicks des Trägers in Fernsicht entspricht; und
c2) ein reduzierter Progressionslängenbereich [Lpmin; Lpmax] wird abhängig vom Vorzeichen der Differenz $\Delta$ bestimmt.

6. Verfahren zur Bestimmung mindestens eines optischen Konzeptionsparameters für eine ophthalmische Gleitsichtlinse nach Anspruch 1, wobei im Schritt c3) ein Progressionslängenwert (Lp) gleich der Montagehöhe (FH, Hd) verringert um eine Menge $\varepsilon$ bestimmt wird, wobei $\varepsilon$ eine Funktion der Differenz $\Delta$, der Montagehöhe (FH, Hd), eines Okularrefraktionsmesswerts des Trägers (Rx) und/oder der Abnutzungszone (ZU) ist.

7. Verfahren zur Bestimmung mindestens eines optischen Konzeptionsparameters für eine ophthalmische Gleitsichtlinse nach Anspruch 1, das außerdem die folgenden Schritte enthält:

b5) mindestens ein für eine Dispersion ($\Delta\beta$, XU, YU) der Abnutzungszone (ZU) repräsentativer Wert wird berechnet;
c5) ein Progressionslängenwert (Lp) wird abhängig vom Montagehöhe-Messwert (FH, Hd), vom Wert der Differenz $\Delta$ und vom mindestens einen für eine Dispersion ($\Delta\beta$ XU, YU) der Abnutzungszone (ZU) repräsentativen Wert bestimmt.

8. Verfahren zur Bestimmung mindestens eines optischen Konzeptionsparameters für eine ophthalmische Gleitsichtlinse nach Anspruch 7, wobei im Schritt c5) ein Progressionslängenwert (Lp) gleich der Montagehöhe (FH, Hd) verringert um eine Korrekturfunktion $\varepsilon$ bestimmt wird, wobei $\varepsilon$ eine Funktion der Differenz $\Delta$, der Montagehöhe (FH, Hd) und der Dispersion der Abnutzungszone (ZU) ist.

9. Verfahren zur Bestimmung mindestens eines optischen Konzeptionsparameters für eine ophthalmische Gleitsichtlinse nach Anspruch 3, das außerdem die folgenden Schritte enthält:

b6) die Position einer Grenze (HU, LU, NU, TU) der Abnutzungszone (ZU) wird berechnet;
c6) ein Progressionsprofil der optischen Leistung gemäß einem Meridian zwischen der Fernsichtzone (11) und der Nahsichtzone (12) wird abhängig von der Position der Grenze (HU, LU, NU, TU) der Abnutzungszone (ZU) bestimmt.

10. Verfahren zur Bestimmung mindestens eines optischen Konzeptionsparameters für eine ophthalmische Gleitsichtlinse nach einem der Ansprüche 2 bis 9, das außerdem die folgenden Schritte enthält:

b7) die Position einer Grenze (NU, TU) der Abnutzungszone (ZU) wird berechnet;
c7) der Wert des inneren Versatzes (E) wird abhängig von der Position der Grenze (NU, TU) der Abnutzungszone (ZU) bestimmt.

11. Verfahren zur Bestimmung mindestens eines optischen Konzeptionsparameters für eine ophthalmische Gleitsichtlinse nach Anspruch 2, das außerdem die folgenden Schritte enthält:

b8) eine senkrechte Ausbreitung (YU) der Abnutzungszone (ZU) wird berechnet;
c8) die Höhe der Nahsichtzone (12) wird abhängig von der Position des Schwerpunkts (BU) der Abnutzungszone (ZU) und der senkrechten Ausbreitung (YU) der Abnutzungszone (ZU) bestimmt.

12. Verfahren zur Bestimmung mindestens eines optischen Konzeptionsparameters für eine ophthalmische Gleitsichtlinse nach einem der Ansprüche 2 oder 11, das außerdem die folgenden Schritte enthält:

b9) eine waagrechte Ausbreitung (XU) der Abnutzungszone (ZU) wird berechnet;
c9) die Breite der Nahsichtzone (12) wird abhängig von der waagrechten Ausbreitung (XU) der Abnutzungszone (ZU) bestimmt.

13. Verfahren zur Bestimmung mindestens eines optischen Konzeptionsparameters für eine ophthalmische Gleitsichtlinse nach einem der Ansprüche 1 bis 12, wobei der optische Konzeptionsparameter abhängig vom sphärischen Kompensationswert (Rx) des Rezepts der ophthalmischen Gleitsichtlinse und/oder vom Wert der optischen Leistungsaddition zwischen der Fernsichtzone (11) und der Nahsichtzone (12) moduliert wird.

14. Verfahren zur Bestimmung mindestens eines optischen Konzeptionsparameters für eine ophthalmische Gleitsichtlinse nach einem der Ansprüche 1 bis 13, wobei der optische Konzeptionsparameter abhängig von einer Messung eines Neigungswinkels des Kopfes des Trägers moduliert wird.

15. Verfahren zur Bestimmung mindestens eines optischen Konzeptionsparameters für eine ophthalmische Gleitsichtlinse nach einem der Ansprüche 1 bis 14, wobei:

d) eine Vielzahl von Mittelwerten bezüglich einer Abnutzungsfläche (ZU) geliefert wird, die einer Vielzahl von Bezugsträgern zugeordnet sind;
e) die der Vielzahl von Bezugsträgern zugeordnete Vielzahl von Mittelwerten statistisch behandelt wird, um eine statistische Verteilung der Vielzahl von Mittelwerten zu bestimmen;
f) ein Mittelwert bezüglich der Abnutzungsfläche (ZU) für einen Träger während der Sehaufgabe bestimmt wird;
g) mindestens ein optischer Konzeptionsparameter für eine ophthalmische Gleitsichtlinse für den Träger abhängig vom Mittelwert bezüglich der Abnutzungsfläche für den Träger und von der statistischen Verteilung von Mittelwerten bestimmt wird, die der Vielzahl von Bezugsträgern zugeordnet sind.

## Claims

1. A method for determining at least one optical conception parameter for a progressive ophthalmic lens intended to equip a frame (10) of a wearer, depending on the visual behavior of the latter, said at least one optical conception parameter comprising a progression length (Lp) of said progressive ophthalmic lens, the method comprising the following steps:

a) collecting data associated with the chosen frame (10) and data relating to the prescription of the wearer, and collecting a plurality of behavioral measurements relating to a plurality of gaze directions and/or positions of the wearer during a visual task; and a3) acquiring a measurement of fitting height (FH, Hd) for said progressive ophthalmic lens in said frame (10);
b) statistically processing said plurality of behavioral measurements in order to determine a zone of use (ZU) of the area of an eyeglass fitted in said frame, said zone of use (ZU) being representative of a statistical spatial distribution of said plurality of behavioral measurements; b1) calculating a vertical position of the centroid (BU) or a high limit (HU) of the zone of use (ZU) and b3) calculating a value of a difference $\Delta$ between the vertical position of the centroid (BU) or the high limit (HU) and a reference vertical position (17), corresponding to a mean drop angle of the gaze relative to a primary direction (D0) of the far-vision gaze of the wearer; and
c) determining at least one optical conception parameter comprising a value of progression length (Lp) for said progressive ophthalmic lens depending on said measurement of fitting height (FH, Hd) and on said difference $\Delta$.

2. The method for determining at least one optical conception parameter for a progressive ophthalmic lens as claimed in claim 1, in which said at least one optical conception parameter comprises at least one optical conception parameter of the design of said progressive ophthalmic lens among a restricted progression-length range [Lpmin; Lpmax], a height of the near-vision zone (12), a width of the near-vision zone (12), and an internal offset (E) of said progressive ophthalmic lens.

3. The method for determining at least one optical conception parameter for a progressive ophthalmic lens as claimed in claim 1 or 2, in which said at least one optical conception parameter comprises a profile of progression of the optical power along a meridian between the far-vision zone (11) and the near-vision zone (12) for said progressive ophthalmic lens.

4. The method for determining at least one optical conception parameter for a progressive ophthalmic lens as claimed in claim 2, furthermore comprising the following steps:
c1) determining a restricted progression-length range [Lpmin; Lpmax] depending on the position of the centroid (BU) of the zone of use (ZU).

5. The method for determining at least one optical conception parameter for a progressive ophthalmic lens as claimed in claim 4, furthermore comprising the following steps:

b2) determining the sign of the difference $\Delta$ between the vertical position of the centroid (BU) and a reference vertical position (17), corresponding to a mean drop angle of the gaze relative to a primary direction (D0) of the far-vision gaze of the wearer; and
c2) determining a restricted progression-length range [Lpmin; Lpmax] depending on the sign of the difference $\Delta$.

6. The method for determining at least one optical conception parameter for a progressive ophthalmic lens as claimed in claim 1,
in which in step c3) a value of progression length (Lp) is determined, said value being equal to the fitting height (FH, Hd) decreased by a quantity $\varepsilon$ where $\varepsilon$ is a function of the difference $\Delta$, of the fitting height (FH, Hd), of a measurement of ocular refraction of the wearer (Rx) and/or of the zone of use (ZU).

7. The method for determining at least one optical conception parameter for a progressive ophthalmic lens as claimed in claim 1, furthermore comprising the following steps:

b5) calculating at least one value representative of a dispersion ($\Delta\beta$, XU, YU) of the zone of use (ZU); and
c5) determining a value of progression length (Lp) depending on said measurement of fitting height (FH, Hd), on said value of the difference $\Delta$ and on said at least one value representative of a dispersion ($\Delta\beta$, XU, YU) of the zone of use (ZU).

8. The method for determining at least one optical conception parameter for a progressive ophthalmic lens as claimed in claim 7, in which in step c5) a value of progression length (Lp) is determined, said value being equal to the fitting height (FH, Hd) decreased by a correction function $\varepsilon$ where $\varepsilon$ is a function of the difference $\Delta$, of the fitting height (FH, Hd) and of the dispersion of the zone of use (ZU).

9. The method for determining at least one optical conception parameter for a progressive ophthalmic lens as claimed in claim 3, furthermore comprising the following steps:

b6) calculating the position of a limit (HU, LU, NU, TU) of the zone of use (ZU); and
c6) determining a progression profile of the optical power along a meridian between the far-vision zone (11) and the near-vision zone (12) depending on the position of said limit (HU, LU, NU, TU) of the zone of use (ZU).

10. The method for determining at least one optical conception parameter for a progressive ophthalmic lens as claimed in one of claims 2 to 9, furthermore comprising the following steps:

b7) calculating the position of a limit (NU, TU) of the zone of use (ZU); and
c7) determining the value of the internal offset (E) depending on the position of said limit (NU, TU) of the zone of use (ZU).

11. The method for determining at least one optical conception parameter for a progressive ophthalmic lens as claimed in claim 2, furthermore comprising the following steps:

b8) calculating a vertical spread (YU) of the zone of use (ZU); and

c8) determining the height of the near-vision zone (12) depending on the position of the centroid (BU) of the zone of use (ZU) and on the vertical spread (YU) of the zone of use (ZU).

12. The method for determining at least one optical conception parameter for a progressive ophthalmic lens as claimed in one of claims 2 or 11, furthermore comprising the following steps:

   b9) calculating a horizontal spread (XU) of the zone of use (ZU); and
   c9) determining the width of the near-vision zone (12) depending on the horizontal spread (XU) of the zone of use (ZU).

13. The method for determining at least one optical conception parameter for a progressive ophthalmic lens as claimed in one of claims 1 to 12, in which the optical conception parameter is modulated depending on the spherical-compensation value (Rx) of the prescription of the progressive ophthalmic lens and/or on the value of the optical-power addition between the far-vision zone (11) and the near-vision zone (12).

14. The method for determining at least one optical conception parameter for a progressive ophthalmic lens as claimed in one of claims 1 to 13, in which the optical conception parameter is modulated depending on a measurement of an angle of inclination of the head of the wearer.

15. The method for determining at least one optical conception parameter for a progressive ophthalmic lens as claimed in one of claims 1 to 14, in which:

   d) a plurality of mean values relating to an area of use (ZU) are provided, said values being associated with a plurality of reference wearers;
   e) said plurality of mean values associated with said plurality of reference wearers are statistically processed in order to determine a statistical distribution of said plurality of mean values;
   f) a mean value relating to the area of use (ZU) is determined for a wearer during said visual task; and
   g) at least one optical conception parameter is determined for a progressive ophthalmic lens for said wearer depending on said mean value relating to the area of use for said wearer and on said statistical distribution of mean values associated with said plurality of reference wearers.

## Fig.1

IVL

11

CM

10

E

y

x

FH

$H_d$

LP

IVP

12

## Fig.2

40

y

x

CRO

IVL

I1

I2

DO

A1

A2

D1

D2

21

C1

C2

20

PM

10

## Fig.3

IVL

11

PM

CM

I1

I3

HU

NU

I4

YU

I2

TU

ZU

LU

BU

XU

12

y

x

Fig.4A

Fig.4B

Fig.5

Fig.6

Fig.7

Fig.8

Fig.9

Fig.10

**Fig.11**

**Fig.12**

**Fig.13**

## Fig.14

## Fig.15A

## Fig.15C

## Fig.15B

## Fig.16

$$LOC = K\beta + C$$

**EP 3 620 101 B1**

## RÉFÉRENCES CITÉES DANS LA DESCRIPTION

### Documents brevets cités dans la description

- US 8297752 B
- US 2003107707 A1
- US 6827443 B2
- FR 2914173